# EUROPEAN PATENT SPECIFICATION

(11) **EP 2 916 720 B1**
(45) Date of publication and mention of the grant of the patent: **08.12.2021**
(21) Application number: 13815577.5
(22) Date of filing: 30.10.2013
(51) Int. Cl.: A61B 5/00, H04B 10/80, H04B 10/40, H04B 10/25, A61B 5/301, A61B 5/318, A61B 1/005, A61B 5/01, A61B 8/12

(54) **AN OPTICAL PROBE SYSTEM**
OPTISCHES SONDENSYSTEM
SYSTÈME DE SONDE OPTIQUE

(30) Priority: 08.11.2012 US 201261723853 P
(43) Date of publication of application: 16.09.2015
(73) Proprietor: Koninklijke Philips N.V., 5656 AG Eindhoven (NL)
(72) Inventor: VAN DER MARK, Martinus Bernardus, NL-5656 AE Eindhoven (NL); VAN DUSSCHOTEN, Anna Hendrika, NL-5656 AE Eindhoven (NL)
(74) Representative: Philips Intellectual Property & Standards
(86) International application number: PCT/IB2013/059794
(87) International publication number: WO 2014/072891

(56) References cited:
- JP-A- S60 209 717
- US-A- 4 417 140
- US-A- 5 453 866
- US-A- 5 528 409
- US-A1- 2002 161 309
- US-A1- 2009 110 148
- US-A1- 2011 044 694
- US-B1- 6 575 965
- SCHUBERT MARTIN ET AL: "Electroluminescence induced by photoluminescence excitation in GaInN/GaN light-emitting diodes", APPLIED PHYSICS LETTERS, AMERICAN INSTITUTE OF PHYSICS, US, vol. 95, no. 19, 10 November 2009 (2009-11-10), pages 191105-191105, XP012126380, ISSN: 0003-6951, DOI: 10.1063/1.3258488 cited in the application

## Description

### FIELD OF THE INVENTION

The present invention relates to an optical probe system, and more particularly relates to an optical probe system comprising an optoelectronic device being arranged for converting a first radiation beam into electrical energy, the invention relates to a corresponding optical probe, and the invention further relates to a corresponding method.

### BACKGROUND OF THE INVENTION

There is a clear and ongoing trend to replace conventional surgical procedures with minimally invasive (MI) interventions. Reduced trauma, shorter hospital stay and reduced cost are the most important drivers of the adoption of minimally invasive techniques. To enable further innovation in medical instrumentation - thus enabling more advanced and more challenging MI interventions - there is a need to integrate miniature sensors for in-body imaging and physiological measurement in instruments like catheters and guide wires.

Data and power delivery to the tip of long and thin devices such as a medical catheter or guide wire for imaging, sensing, sensitising or even ablation can be challenging. Including, on top of that, a high data rate return channel is even more problematic. This is due to several reasons.

Firstly, the combination of the small cross section (i.e. small diameter), necessary for the medical intervention, combined with the long length of a guide wire or catheter does severely limit the total number of electrical wires that can be integrated in such an instrument.

Secondly, the integration of (multiple) electrical wires compromises the flexibility of the instrument, while flexibility is a key property of this type of instruments.

Thirdly, for high data rate, such as e.g. required for an ultrasound transducer at the tip or sensitive measurements, one often requires coaxial cables which need even more space compared to single-core wires.

Fourthly, instruments with electrical wires typically are not compatible with the use of MRI due to resonances in/of the electric wiring leading to electromagnetic interference in the connected electronics and also possibly leading to tissue heating. And furthermore, thin electrical cables typically cannot support a relatively high amount of power for use at the distal end of the catheter.

Also, because of their disposable use, catheters and guide wires must be manufactured in a relatively simple and cost effective way.

US patent 7,831,152 discloses an optical transceiver for detecting an incoming light beam and for transmitting an outgoing light beam along a common optical axis, the outgoing light beam may contain control or information signals. Such an optical transceiver provides a compact transceiver that is suitable for a wide variety of applications, for example a catheter or other kind of probes. In some embodiments, optical power is provided to an optical detector, which is converted into electrical energy for use at the distal end of an optical probe.

One disadvantage of this device is for example the use of a special multiple junction and/or stacked optoelectronic device. The device is furthermore GaAs/AlGaAs-based, hence it has a relatively low bandgap, and therefore the voltage produced is too low to power silicon-based electronics with only one junction. Further, the toxicity of As may be an issue for the use in medical devices.

US 5453866, according to its abstract, relates to a measurement system that uses optical energy for a sensor and analog optical transmission of the measuring signals of the sensor. A feedback control system sets the working point of the optical transmission system for the measuring signals. A zero measuring signal is provided to an electro-optical signal converter, which outputs a corresponding optical analog signal via an optical transmission line to an optoelectric signal converter. The optoelectric signal converter is adjusted to a predefined analysis signal setpoint when the electro-optical signal converter receives the zero measuring signal via a feedback control system. The manipulated variable of the feedback control system is the working-point current of the electro-optical signal converter, which converts the electrical measuring signals into optical signals. This feedback control system can be used to compensate for interference in the analog transmission. An additional feedback control system, which amplifies the analysis signals into output signals, is provided to correct the gain factor of an amplifier unit.

US 2009/0110148 A1, according to its abstract, relates to a device interface selectively acquires patient physiological parameter data. An acquisition processor acquires physiological data from a patient. A communication processor is coupled to an optical communication pathway for receiving a plurality of optical signals from a source. A conversion processor is electrically coupled to the acquisition processor and communication processor and converts a first optical power signal at a first frequency and received via the optical communication pathway using the communication processor, to a first electrical signal for providing power to said device interface. The conversion processor converts an optical control signal at a second frequency different from the first frequency and received via the optical communication pathway using the communication processor, to a second electrical signal for providing control data to the acquisition processor directing the acquisition processor to acquire at least one physiological parameter from a patient.

US 5528409, according to its abstract, relates to a fiber-optic data-link used to interface a remote process variable sensor/transmitter to a local process control system that includes a local light source module and a remote interface module interconnected by one or more optical fibers. Energy for operating the remote interface module and its associated process variable sensor/transmitter is provided by light energy transmitted from the local site to the remote site and converted at the remote site to an electrical signal. The local light source module and the remote interface module each may include a microcontroller. The controllers not only control the flow of status messages in either analog or digital or analog and digital form simultaneously from the remote site to the local site, but also insure the integrity of the system and minimizes the amount of optical power delivered to the remote site to that which is required to electrically power the remote interface module and the process variable sensor/transmitter, thus extending the useful life of the light source. The system is also arranged to check the integrity of the optical fibers to ensure that the light intensity is always below a minimum eye-safe value whenever the optical fiber is not correctly connected between the local light source module and the remote interface module.

US 2002/0161309 A1, according to its abstract, relates to a reusable appliance for acquisition from a patient of EEG signals comprising a micro-miniature, micro-power, low noise multi-channel data-acquisition system powered by a fiber optic illuminator and photovoltaic cell thus eliminating the need for inductively coupled power converters. The appliance also uses disposable EEG electrodes which are similar in size and very low cost.

US 4417140, according to its abstract, relates to a fiber-optically coupled measuring device which has an optically energized transducer section which produces signal information in an optical form, the energy supply for the transducer section and the signal information therefrom being transmitted via a light guide fiber. The signal information is transmitted to a receiver at a location remote from the transducer section, the signal information being modified in response to electrical signals fed to a light emitting component of the transducer section.

The inventors of the present invention have appreciated that an improved optical probe system is of benefit, and have in consequence devised the present invention.

### SUMMARY OF THE INVENTION

It would be advantageous to achieve an improved optical probe system. It would also be desirable to enable an optical probe system working faster and/or more accurate, particularly with a higher data transmission and/or a relatively higher power provided at the distal end of the probe.

The object of the present invention is solved by the subject-matter of the independent claims; further embodiments are incorporated in the dependent claims.

In general, the invention preferably seeks to mitigate, alleviate or eliminate one or more of the above-mentioned disadvantages singly or in any combination. In particular, it may be seen as an object of the present invention to provide a system and a method that solves the above mentioned problems, or other problems, of the prior art. Other problems may be efficiency and/or high power density to allow for a small optoelectronic device at the tip of a catheter/probe so that the invasive medical device remains relatively thin and so that tissue heating is avoided and/or minimized as well.

To better address one or more of these concerns, in a first aspect of the invention an optical probe according to claim 1 is provided.

The invention is particularly, but not exclusively, advantageous for obtaining an improved optical probe system capable of obtaining a higher data transmission and/or a relatively high power at the distal end of the optical probe system with relatively high efficiency.

Many simultaneous advantages may also come from using an optical guide, e.g. an optical fiber, directly connected to a bi-directional opto-electronic device at the tip of an elongated instrument. This may be true in particular in the medical domain for guide wires or catheters, though the present invention may also find application in other areas where optical probes may be beneficially used.

It is proposed to use an optical fiber linked to, at the distal end, a two-way opto-electronic device which is addressed and powered from the proximal end solely by light launched into and guided by the optical guide or optical fiber.

Typical examples of two-way opto-electronic devices may be semiconductor light emitters: they can be used as photodetectors as well. Two-way opto-electronic devices are, for example: LEDs, RCLEDs (resonant cavity light emitting diodes), semiconductor lasers or VCSELs (vertical-cavity surface-emitting lasers). Note that all those devices are made from direct band gap materials, though the present invention may also be implemented with indirect bandgap materials.

Another advantage may be seem from the facts that temperature increase of tissue near the distal end of the probe, and hence power load on the tissue should be limited. This is to prevent heating of the tissue to a temperature higher than the denaturation temperature of 42 °C. This may require, amongst other factors:
- efficient power conversion at the tip of a catheter or guide wire.
   and/or
- cooling of the tip by flow of water or another coolant through the catheter. This will allow much higher power to be delivered to the tip of the instrument in order to compensate for losses in the optoelectronics and/or electronics, within the catheter or on the interface between catheter and tissue. However, this solution may compromise the lateral space available in instrument.

It should be noted that, in the context of the present invention, lasers or power-LEDs are relatively ideal photovoltaic converters due to their low intrinsic series resistivity as well as for their optimization for a narrow wavelength band. Both LEDs and similar lasers typically produce substantial current when illuminated with light of preferably slightly shorter wavelength (for example 2-20%, such as 5-10%) than under their normal operating conditions when they produce light. It appears that a combination of a BluRay Disk violet laser at 405 nm works well with some high power blue LEDs from Philips Lumileds or Osram, normally operating at 440-450 nm. Currently, high power LEDs or lasers are particularly suitable for high-power and high power density photo voltaic conversion. It is conceivable that new, dedicated and even more optimal photovoltaic conversion devices will be designed in the future that may particularly be implemented when considering the general principle and teaching of the present invention.

Furthermore, it should be noted that when illuminating a LED (or Laser) it is possible to produce so-called photo-induced electroluminescence (PIEL), see for example F. Schubert, Q. Dai, J. Xu, J. K. Kim and F. Schubert, "Electroluminescence induced by photoluminescence excitation in GaInN/GaN light-emitting diodes", Applied Physics Letters, Volume 95, 191105 (2009). This reference explains the physical principle behind photo-induced electroluminescence that may beneficially be applied in optical probes according to the present invention. The intensity of this PIEL luminescence typically depends on the electrical load on the LED device's terminals, and hence it can be modulated by the electronics at the distal end without the need for electrical power for the LED device, which would require a voltage higher than the bandgap to overcome resistive losses in the LED and driving circuit. In the situation sketched, the power comes directly from the laser or the radiation source at the proximal end and less resistive loss (with the associated drop in voltage) will normally occur. This provides an excellent way for a data return channel using a single device, that is, the present invention enables optical data multiplexing in a new and advantageous manner. In case of a power LED, such as the Philips Lumileds Luxeon Rebel, the bandwidth will be on the order of 1 MHz. In case a laser is used as 2-way optoelectronic device is at the distal end, the bandwidth is expected to be on the order of 400 MHz, or even higher.

An additional advantage of an optical link for data communication provided by the present application is its MRI compatibility and total electrical insulation of proximal and distal end. Note also that the electrical resistance of the body itself provides a natural lower bound on practical values of electrical insulation.

An extended application may also exploit the combination of using a limited number (e.g. 2) of (thin, highly resistive) electrical wires to deliver a high-voltage for biasing (larger than a few volts, typically 100 Volt) to the sensor device at the tip (for example a CMUT ultrasound receiver or transducer), while the optical link is used for power and to program and control the sensor device (for example a data multiplexer) and transfer the data. In one embodiment, the electrical impedance of the high-voltage part of the electrical circuit can be large (i.e. a capacitor with a (quasi-)static bias voltage or a high-impedance resistor), in which case the resistance of the electrical wire can also be chosen large, which improves the MR-compatibility of this solution compared to an all-electrical solution. In a preferred embodiment, the highly resistive electrical connection can be an integral part of the coating of the optical fiber, or even the coating itself. A typical resistance would be 1MΩ.

The present invention may particularly be implemented together with optical shape sensing fibers for use in medical applications, or other similar applications. The optical shape sensing may be based on either Fiber Bragg Gratings (FBG) or Rayleigh backscattering in the optical guide or fiber.

In the context of the present application, it is to be understood that an optical probe may, but not exclusively, be considered as an elongated, or extended, shape with a proximal end and a distal end, the latter being used for monitoring and/or various applications, e.g. RF ablation and ultrasonic purposes.

In the context of the present application, it is to be understood that a radiation source may include any suitable transmitter of electromagnetic radiation, for example infrared light (IR), visible light, ultraviolet light (UV), X-Ray radiation, etc.

In an embodiment, the optical guide is arranged for guiding said first radiation beam from the proximal end to the distal end, and further being arranged for guiding said second radiation beam from the distal end to the proximal end, the first radiation beam and the second radiation beam being arranged for being guided along the same optical path, or a parallel optical path, in said optical guide. A possible advantage may be that only a single guide is needed. In a particular embodiment, the optical guide may have one or more optical channels.

The optical guide may be arranged for allowing transmission of radiation in single mode. In another possible embodiment, the optical guide may be arranged for allowing transmission of radiation in multimode. The optical guide may be manufactured in a single optical material. The optical guide may have multiple cores, such as comprising a multicore fiber, wherein each core may be individually prepared as it is well-known to the skilled person in optics.

In another embodiment, the optical guide comprises an optical fiber, the optical fiber comprising at least a part of the said optical path for the first radiation beam and the second radiation beam. In a particular configuration, there is provided an optical guide comprising an optical fiber with separate optical cores.

In another embodiment, the system further comprises a control unit (CON), the control unit being operably connected to the radiation source and arranged for controlling the optical energy and providing the first data thereto, the control unit further being operably connected to the photodetector and arranged for receiving the second data therefrom. This may be advantageous because an improved control system in a single unit is provided.

In another embodiment, the control unit is configured for operating a control loop for controlling the optical energy (O_P) and/or the first data (D_F) based, at least partly, on the second data (D_R). This may be advantageous since feedback may be embedded in the second data, for example power deliver response of tissue to power delivered there to. This may be particularly important when used in humans because automatic control can avoid or minimize unintended damages.

In another embodiment, said second radiation beam is dependent upon an electrical load on the optoelectronic device. This may advantageously provide an efficient multiplexing data channel capable of providing high data transfer in both directions. In a specific embodiment, the effect of photo-induced electroluminescence (PIEL) is utilized.

In another embodiment, said control loop is arranged for optimizing the electrical load on the optoelectronic device. This may be beneficial because it is possible to find the maximum power point (MPP), where the maximum power point is the optimum point of operating the system, in particularly the optimum point of operating the optoelectronic device.

In another embodiment, the optoelectronic device comprises a photovoltaic converter, preferably the optoelectronic device comprises a solid-state laser, or a light emitting diode (LED). This may be beneficial because it might be possible to find the maximum power point (MPP), where the maximum power point is the optimum point of operating the system, in particularly the optoelectronic device. This is explained further in the detailed description of the invention.

In another embodiment, the optoelectronic device is a direct band-gap device, preferably a single junction device where
1) the converting of said first radiation beam into electrical energy and receiving said first data, and
2) the emitting of said second radiation beam towards the photodetector, said emission being inducible by the incoming first radiation beam, such as said emission being photo-inducible by the incoming first radiation beam, is arranged for taking place at said single junction. A junction may be broadly defined, but not necessarily limited to, an interface between two, or more, distinct materials which defines an active region capable of facilitating optical and/or opto-electrical phenomenons. The single junction may for example be a p-n junction in the optoelectronic device at which all conversion takes place: power, data and luminescence.

According to the invention, the optoelectronic device is capable of performing photo-induced electroluminescence (PIEL). This may be particularly advantageous because relatively fast two-way optical communication may be provided.

In another embodiment, the optical probe, at its proximal end, comprises an optical element capable of separating the first and the second radiation beam, wherein the optical element may for instance be a semi-transparent or dichroic mirror.

In another embodiment, the optical converter circuit is powerable solely by said electric energy from the optoelectronic device. This may be advantageous because electric wiring to the distal end may not be needed to the same extent, such as may be avoided or minimized.

In another embodiment, the optical converter circuit is powerable directly by said electric energy from the optoelectronic device without any voltage up up-conversion. For example, the optical converter circuit may comprise a GaN based device such as a photodiode, LED or diode laser facilitating that the optoelectronic device is capable of driving silicon based electronics requiring approximately 2 Volt, hence no voltage up-conversion is necessary.

In another embodiment, the application device is controllable in response to the first data. In another embodiment, the application device comprises any one of:
- a temperature sensor,
- a pressure sensor,
- a chemical sensor,
- an ultrasound transducer (CMUT),
- a camera,
- a sensor for ionizing radiation (alpha, beta and/or gamma),
- an electric field sensor for example for measuring an ECG (electrocardiogram),
   and/or
- an electric stimulator or sensitizer.

In another embodiment, the optical probe system may be used for optical shape sensing.

The skilled person readily understands that other devices may be implemented. It is noted that the probe system may be powered optically but the application device may be non-optically-based, for example by using ultrasound, voltammetry, strain gauges or other non-optical principles, techniques or modalities.

According to a further aspect, the invention relates to a method for supplying an optical probe with electrical energy and for sending and receiving data from the optical probe according to claim 14.

Other aspects, features and/or advantages will be apparent from and elucidated with reference to the embodiments described hereinafter.

### BRIEF DESCRIPTION OF THE DRAWINGS

Embodiments of the invention will be described, by way of example only, with reference to the drawings, in which
FIG. 1 shows a schematic embodiment of an optical probe system according to the present invention,
FIG. 2 shows a graph of power load curves (left axis) as a function of photo-induced (or photo-voltaic) current through the LED for 4 different currents through the driving laser according to the present invention. It also shows, on the right axis, the amount of photo-induced electroluminescence,
FIG. 3 shows a graph with laser power versus laser current (left axis). On the right axis, photo-luminescence and photo-induced electroluminescence of the laserilluminated LED are given, for the corresponding laser power,
FIG. 4 shows another graph with load curves for two different LEDs, top curve: LD G5AP (OSRAM) with a surface area of 0.4×0.4 mm², and bottom curve: Luxeon Rebel Royal Blue (LUMILEDS) with a surface area of 1.0×1.0 mm², and
FIG. 5 shows a flow chart of a method according to the present invention.

### DESCRIPTION OF EMBODIMENTS

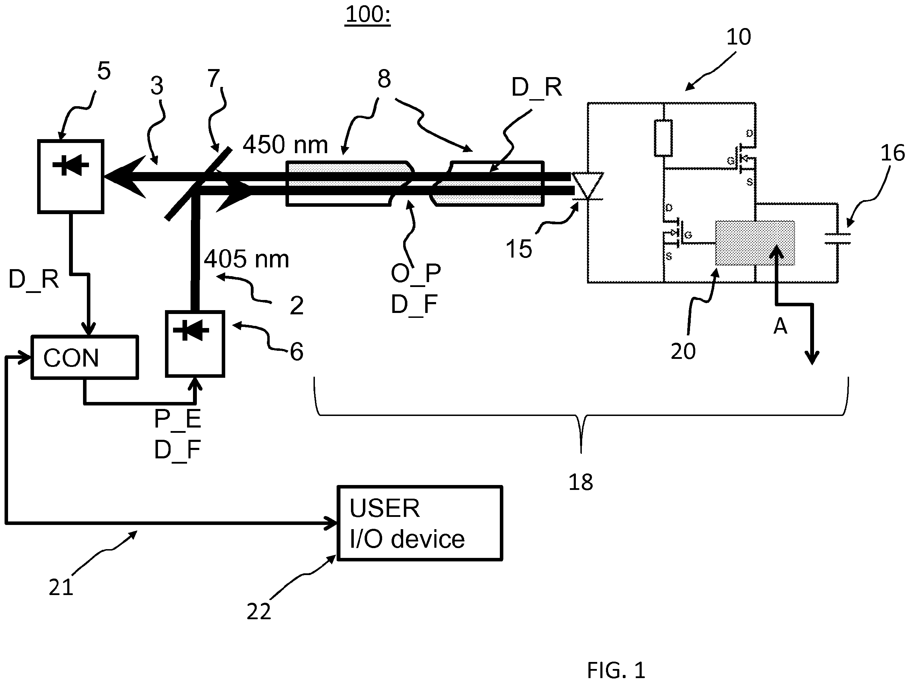

FIG. 1 shows a schematic embodiment of an optical probe system 100 according to the present invention.

A radiation source 6 is capable of emitting a first radiation beam, said first radiation beam 2 comprising optical energy O_P and first data D_F. A photodetector 5, the photodetector is arranged for detecting a second radiation beam 3.

An optical probe is, at its proximal end, optically connected to the photodetector and the radiation source, the probe having an optical guide 8, for example an optical fiber as schematically illustrated in FIG. 1, capable of connecting the distal end with the proximal end, the optical probe having at its distal end an optical converter circuit 10, the circuit comprising:
An application device 20, the application device being arranged for monitoring and/or manipulation, e.g. the influencing surrounding tissue of a human with acoustic or electromagnetic radiation, at the distal end of the probe, the application device being arranged for generating second data D_R indicative of the functionality of the application device.
An optoelectronic device 15, the optoelectronic device being arranged for converting said first radiation beam 2 into electrical energy and for receiving said first data, the first data being related to the functionality of the application device, the optoelectronic device further being arranged for emitting said second radiation beam 3 towards the photodetector, the second radiation beam comprising the second data,
   the optoelectronic device further being arranged for:
      - converting said first radiation beam into electrical energy and for receiving said first data, and
      - emitting said second radiation beam towards the photodetector, said emission being inducible by the incoming first radiation beam,
   wherein the optical converter circuit 10 is powerable by said electric energy in the first radiation beam 2. Thus, the optical energy O P, originated from radiation source 6 being powered via controller CON delivering energy P_E to it, is radiated to the circuit 10 at the distal end and there converted into electrical energy useable in the circuit.

In this embodiment, optical power is delivered through the fiber 8 by a shortwavelength laser 6, positioned at the proximal end of the fiber, which is integrated in a medical device such as a catheter or guide wire 18. A high band-gap photovoltaic receiver may act as the optoelectronic device 15 and thereby provide power for at the tip (distal end) of the fiber.

An example of an embodiment of the power and data delivery system that can be integrated into a catheter or guide wire 18. Light of 405 nm BluRay disk laser 6 is launched into an optical fiber via a dichroic mirror 7. This light illuminates an LED with slightly lower band-gap which as a consequence produces a current in the circuit 10 attached to it. This circuit is designed so that it can influence the electrical impedance felt by the LED. In the example of this figure, it can essentially disconnect the diode by raising the impedance substantially, thereby also disconnecting its own power source. A capacitor 16 is used to bridge the time period without external supply. By disconnecting the diode, photo-induced electroluminescence (PIEL) will occur with a longer wavelength (for example 450 nm) than the absorbed light from the laser (for example 405 nm), and it is collected by the fiber and transported to a detector via the dichroic mirror 7. By this means the circuit 10 can send data D_R back to the control unit CON, and in turn a user 22, e.g. an I/O device, via connection 21 (wireless or dedicated wire (s)).

The advantages results from one, or more, of the following elements:
- single junction optoelectronic device (not stacked, not arranged in series) with 3-way use: simultaneous data in, data out (transceiver for duplexing) and power input
- GaN/AlGaN based, with direct, high band gap typically supplying 2-2.5 Volts
- directly powering silicon based electronics (Voltage required >1.65 Volt)
- small, high power and power density capability > 1W/mm² up to 1kW/mm² or even up to 1MW/mm²
- GaN is non-toxic, an advantage for in-body use
- PIEL is a signature of high efficiency, which in itself makes low heat load on tissue possible.
- Feedback and control loop using PIEL, and/or
- Data return using PIEL

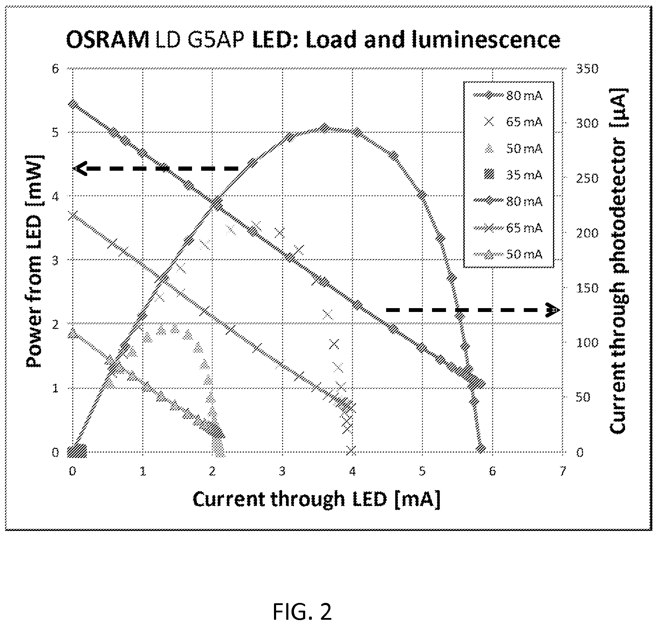

The power and data delivery system 100 can be integrated into a catheter or guide wire 18 is shown in FIG. 1. By this means the circuit can send data. The input power from the laser can be changed to adjust to find the optimum working point for power efficiency (the Maximum Power Point, MPP). A feedback loop can be made in which the total luminescence (the sum of photoluminescence and photo-induced electroluminescence) is monitored to measure the load on the LED and from that to deduce the working point and power efficiency of the electronic circuit. This is illustrated in FIG. 2, which shows a graph with power load curves as a function of current through the LED for 4 different currents through the driving laser.

Additionally, a data stream can modulate the input power on the laser to send data to the circuit. The circuit senses the associated modulation of the supply voltage within a given frequency band. This frequency band presumably lies at higher frequencies than that of the power adjustment control loop.

FIG. 2 shows a graph of power load curves as a function of current through the LED for 4 different currents through the driving laser according to the present invention; Measured data on an LED of type LD G5AP (OSRAM) with a surface area of 0.4×0.4 mm². The graph shows power load curves as a function of current through the LED for 4 different currents through the driving laser. Clearly, the power that can be draw from the LED shows a maximum, this is the so-called maximum power point (MPP). The down-sloping lines belong to the right axis and correspond to the luminescence signal on a collecting photodetector, with the optics configured much like shown in Fig. 1, but with a lens replacing the optical fiber.

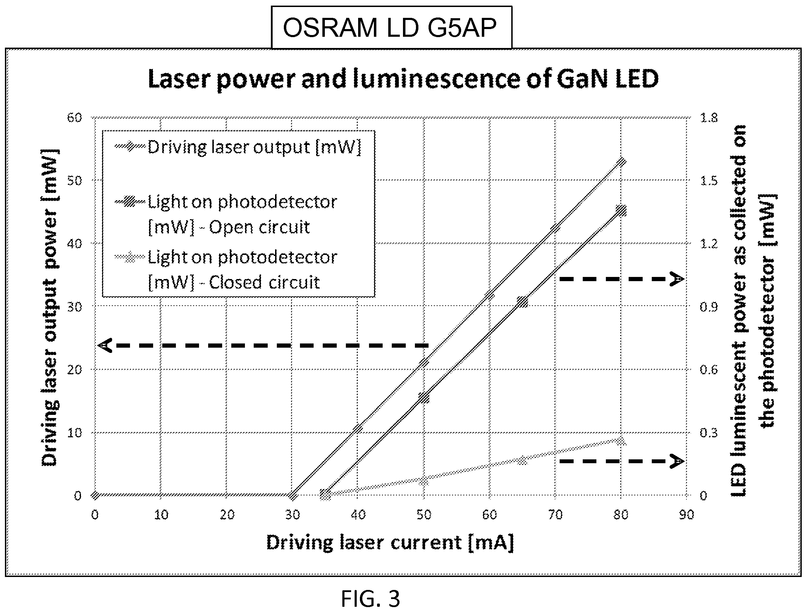

Comparing to FIG. 3 teaches us that the photovoltaic conversion efficiency in the MPP for this LED is approximately 10%. One can also see that the increase in output power is linear with input power; hence power density is not an issue at those power levels.

FIG. 3 shows a graph with laser power versus laser current. In FIG. 3, on the left axis, laser power versus laser current is shown; laser threshold is approximately 30 mA. The right axis gives the collected luminescent power from the OSRAM LED on the photodetector. Only a limited fraction of luminescence was collected. The power at closed circuit represents pure photoluminescence, the power at open circuit the total luminescence. The power was calculated using a photosensitivity of 0.234 A/W at 450 nm of the silicon detector and a load of 50 Ohm.

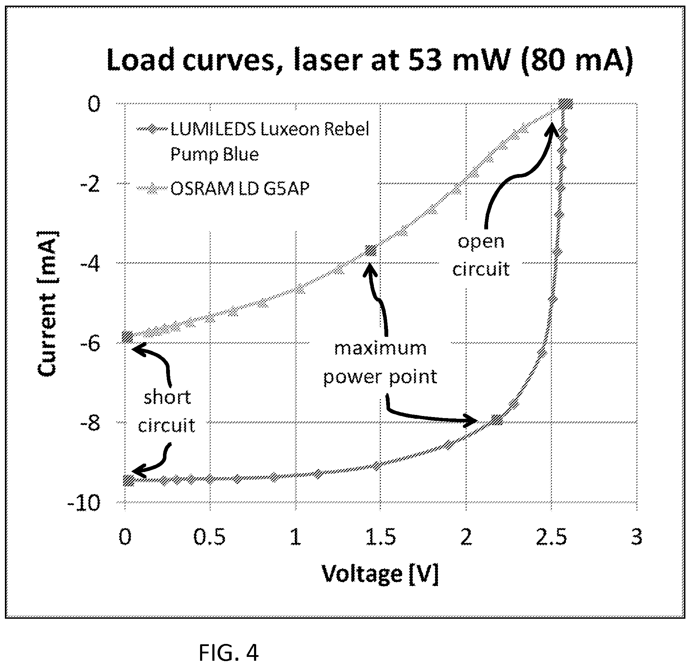

FIG. 4 shows another graph with load curves for two different LEDs. Thus, in FIG. 4, the load curve for two different LEDs is shown. The top curve shows the LD G5AP (OSRAM) with a surface area of 0.4×0.4 mm², and the bottom curve the Luxeon Rebel Royal Blue (LUMILEDS) with a surface area of 1.0×1.0 mm². Input power is 53 mW at a wavelength of 405 nm. The graph shows the maximum power points for each curve which correspond to 10% and 33% power efficiency respectively. With 33% conversion and 17.5 mW power, the LUMILEDS device seems rather suitable for power conversion.

It should be noted that the speed of modulation of a large area power LED is currently limited to the 100 kHz - 1 MHz range. Future developments may or may not change this. The use of a diode laser, such as used as light source for optical recording purposes may improve this to the 100 MHz -1 GHz range. Alternatively, if a low modulation speed LED is used as power converter and receiver of the first data, a second device may be employed for transmitting the second data. For high speed communication, a VCSEL operating at for example 850 nm would do fine; the operating voltage and current are well within the scope of what has been described above and the data rate would increase to 10 Gb/s (Philips ULM Photonics makes such device and it is also relatively small). Again, the great disadvantage would be the position and alignment of the devices, the VCSEL and LED.

The optimum wavelength of power delivery depends on the band gap of the semiconductor. Roughly, the wavelength for power delivery should be about 5-20% shorter than the emission wavelength used for data transmission. The higher the band gap of the semiconductor, the shorter the emission and power-up wavelengths, and the higher the output voltage will be.

A high band gap (2.26 eV) semiconductor like Gallium phosphide (GaP) which emits at the green wavelength of 555 nm has optimum photovoltaic sensitivity at about 440 nm. Power delivery could be realized with, for example, a blue laser at 405 nm. Gallium nitride (GaN) has a band gap of 3.4 eV. Both GaP and GaN provide the potential to deliver power at directly useful voltages to drive Si-based electronics. Note that the maximum voltage *V* that might be produced by the LED in photovoltaic mode is related to its emission wavelength *λ* by *V* = 1.24×10⁻⁶/*λ* . However, in practice this voltage will not be available, rather this equation gives the maximum voltage at 100% quantum efficiency (the inverse, S = *V*⁻¹, is called the photo sensitivity). There are at least three reasons why this voltage will not be obtained in a practical situation:
1) Built-in potential (typically less favourable for materials with an indirect bandgap such as Si (1.12 eV), GaP (2.26 eV). Materials with a direct bandgap are for example GaAs (1.424 eV), InP (1.344 eV), GaN (3.4 eV), and also in particular Ga_{0.5}In_{0.5}P which is used for red emitting lasers (650 nm) and RCLEDs or VCSELs as well as the high-energy junction on double and triple junction photovoltaic cells.
2) Photo absorption depth, this is directly related to the quantum efficiency. Typically, if one irradiates a photodiode at a wavelength just above the band gap, the absorption length in the material will be larger than the depletion depth, and hence many charge carriers will be lost, or, if total material thickness is small, the light will not be absorbed at all
3) Forward leakage due to voltage built up and other losses in the diode (see below for an electronic model using the equivalent circuit).

Instead of using the indirect band-gap material like Si, or better, GaP (at 2.26 eV corresponding to 555 nm), it is possible and favourable to use the direct band-gap material AlGaInP. Note that the latter has a transition to an indirect band gap at 555 nm. One finds that blue 470 nm LEDs made of GaInN/GaN seem to perform very well: the forward voltage (2.75 V at 1 mA) approaches the emission energy (2.64 eV) closely.

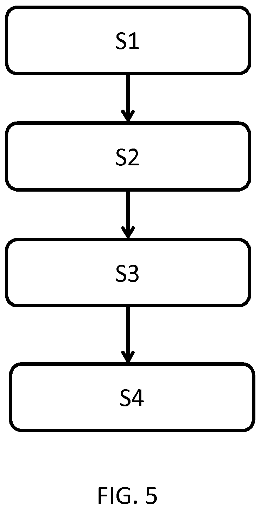

FIG.5 shows a flow chart of a method according to the present invention, more particularly the figure shows a method for supplying an optical probe with electrical energy and for sending and receiving data from the optical probe, the method comprising:
- providing (**S1**) an optical probe system according to the first aspect, said method further comprising:
- emitting (**S2**) a first radiation beam from the radiation source 6, said first radiation beam 2 comprising optical energy O_P and first data D_F,
- converting (**S3**) at the optoelectronic device 15 said first radiation beam into electrical energy and receiving said first data,
- emitting (**S4**) said second radiation beam from the optoelectronic device 15 towards the photodetector.

To sum up, the present description relates to an optical probe system 100 comprising an optical probe 18 having an optical converter circuit 10 with an optoelectronic device 15. The optoelectronic device is arranged for converting a first radiation beam 2 from a radiation source 6 into electrical energy and for receiving first data comprised in said first radiation beam. The optical converter circuit 10 is powerable by said electric energy in the first radiation beam 2. The optoelectronic device is further arranged for emitting a second radiation beam 3 towards a photodetector 5, said emission being inducible by the incoming first radiation beam, the second radiation beam comprising second data. The invention is advantageous for obtaining an improved optical probe system capable of obtaining a higher data transmission and/or a relatively high power at the distal end of the optical probe system with relatively high efficiency and simultaneous at small size.

The illustration and description are to be considered illustrative or exemplary and not restrictive; the invention is not limited to the disclosed embodiments. Other variations can be understood and effected by those skilled in the art from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope. The invention is defined in the appended claims.

## Claims

1. An optical probe (18), the optical probe being at its proximal end optically connectable to an associated photodetector and an associated radiation source, the probe having an optical guide (8) capable of connecting the distal end with the proximal end, the optical probe having at its distal end an optical converter circuit (10), said circuit comprising:
- an application device (20), the application device being arranged for monitoring and/or manipulation at the distal end of the probe, the application device being arranged for generating second data (D_R) indicative of the functionality of the application device, and
- an optoelectronic device (15), the optoelectronic device being arranged for converting a first radiation beam (2) into electrical energy and for receiving first data comprised in the first radiation beam, the first data being related to the functionality of the application device, the optoelectronic device further being arranged for emitting a second radiation beam (3) towards the associated photodetector, the second radiation beam comprising the second data,
the optoelectronic device further having the capability of, upon receiving said first radiation beam:
- converting said first radiation beam into electrical energy and for receiving said first data, and
- emitting said second radiation beam towards the associated photodetector, said emission being inducible by the incoming first radiation beam,
wherein the optical converter circuit (10) is powered by said electric energy in the first radiation beam (2) and the optoelectronic device is configured to perform photo-induced electroluminescence (PIEL).

2. An optical probe system (100), the system comprising:
- a radiation source (6) capable of emitting a first radiation beam, said first radiation beam (2) comprising optical energy (O_P) and first data (D_F),
- a photodetector (5), the photodetector being arranged for detecting a second radiation beam (3), and
- an optical probe (18) according to claim 1,
wherein the optical probe being at its proximal end optically connected to the photodetector and the radiation source.

3. The system according to claim 2, wherein the optical guide (8) is arranged for guiding said first radiation beam (2) from the proximal end to the distal end, and further being arranged for guiding said second radiation beam (3) from the distal end to the proximal end, the first radiation beam and the second radiation beam being arranged for being guided along the same optical path, or a parallel optical path, in said optical guide.

4. The system according to claim 3, wherein the optical guide comprises an optical fiber, the optical fiber comprising at least a part of the said optical path for the first radiation beam and the second radiation beam.

5. The system according to claim 2, wherein the system further comprises a control unit (CON), the control unit being operably connected to the radiation source and arranged for controlling the optical energy and providing the first data thereto, the control unit further being operably connected to the photodetector and arranged for receiving the second data therefrom.

6. The system according to claim 5, wherein the control unit is configured for operating a control loop for controlling the optical energy (O_P) and/or the first data (D_F) based, at least partly, on the second data (D_F).

7. The system according to claim 2, wherein said second radiation beam (3) is dependent upon an electrical load on the optoelectronic device (15).

8. The system according to claim 5 and 7, wherein said control loop is arranged for optimizing the electrical load on the optoelectronic device (15).

9. The system according to claim 2, wherein the optoelectronic device (15) comprises a photovoltaic converter, preferably the optoelectronic device comprises a solid-state laser, or a light emitting diode (LED).

10. The system according to claim 2 or 9, wherein the optoelectronic device is a direct band-gap device, preferably a single junction device, where
1) the converting of said first radiation beam into electrical energy and receiving said first data, and
2) the emitting of said second radiation beam towards the photodetector, said emission being inducible by the incoming first radiation beam, is arranged for taking place at said single junction.

11. The system according to claim 2, wherein the optical converter circuit is powerable solely by said electric energy from the optoelectronic device.

12. The system according to claim 2 or 11, wherein the optical converter circuit is powerable directly by said electric energy from the optoelectronic device without any voltage up up-conversion.

13. The system according to claim 2, wherein the application device comprises any one of:
- a temperature sensor,
- a pressure sensor,
- a chemical sensor,
- an ultrasound transducer (CMUT),
- a camera,
- a sensor for ionizing radiation (alpha, beta and/or gamma),
- an electric field sensor for example for measuring an ECG (electrocardiogram), and/or
- an electric stimulator or sensitizer.

14. A method for supplying an optical probe with electrical energy and for sending and receiving data from the optical probe, the method comprising:
- providing (S1) an optical probe system according to any one of claims 2-13, said method further comprising:
- emitting (S2) a first radiation beam from the radiation source (6), said first radiation beam (2) comprising optical energy (O_P) and first data (D_F),
- converting (S3) at the optoelectronic device (15) said first radiation beam into electrical energy and receiving said first data,
- emitting (S4) said second radiation beam from the optoelectronic device (15) towards the photodetector.

## Patentansprüche

1. Eine optische Sonde (18), wobei die optische Sonde am proximalen Ende optisch mit einem zugehörigen Photodetektor und einer zugehörigen Strahlungsquelle verbunden werden kann, wobei die Sonde eine optische Führung (8) aufweist, mit der das distale Ende mit dem proximalen Ende verbunden werden kann, wobei die optische Sonde am distalen Ende eine optische Wandlerschaltung (10) umfasst, wobei die Schaltung Folgendes umfasst:
- eine Vorrichtung (20), zum Überwachen und/oder Bedienen am distalen Ende der Sonde,
wobei die Vorrichtung Gerät zweite Daten (D_R) generiert, die die Funktionalität des Geräts angeben, und
- ein optoelektronisches Gerät (15), wobei das optoelektronische Gerät ein erstes Strahlenbündel (2) in elektrische Energie umwandelt sowie erste Daten empfängt, die im ersten Strahlenbündel enthalten sind, wobei sich die ersten Daten auf die Funktionalität des Geräts beziehen, und wobei das optoelektronische Gerät zudem ein zweites Strahlenbündel (3) in Richtung des zugehörigen Photodetektors emittiert,
wobei das zweite Strahlenbündel die zweiten Daten enthält,
wobei das optoelektronische Gerät beim Empfangen des ersten Strahlenbündels zudem folgende Schritte durchführt:
- Umwandeln des ersten Strahlenbündels in elektrische Energie sowie Empfangen der ersten Daten und
- Emittieren des zweiten Strahlenbündels in Richtung des zugehörigen Photodetektors,
wobei die Emission durch das eingehende erste Strahlenbündel induziert wird,
wobei die optische Wandlerschaltung (10) mit der elektrischen Energie des ersten Strahlenbündels (2) gespeist wird, und
wobei das optoelektronische Gerät eine photoinduzierte Elektrolumineszenz (PIEL) ausführt.

2. Ein optisches Sondensystem (100), wobei das System Folgendes umfasst:
- eine Strahlungsquelle (6) zum Emittieren eins ersten Strahlenbündels, wobei das erste Strahlenbündel (2) optische Energie (O_P) und erste Daten (D_F) enthält,
- einen Photodetektor (5) zum Erkennen eines zweiten Strahlenbündels (3), und
- eine optische Sonde (18) gemäß Anspruch 1,
wobei die optische Sonde am proximalen Ende optisch mit dem Photodetektor und der Strahlungsquelle verbunden ist.

3. Das System gemäß Anspruch 2, wobei die optische Führung (8) das erste Strahlenbündel (2) vom proximalen Ende zum distalen Ende und zudem das zweite Strahlenbündel (3) vom distalen Ende zum proximalen Ende leitet, wobei das erste Strahlenbündel und das zweite Strahlenbündel entlang desselben optischen Pfads oder eines parallelen optischen Pfads der optischen Führung geleitet werden.

4. Das System gemäß Anspruch 3, wobei die optische Führung einen Lichtwellenleiter umfasst, wobei der Lichtwellenleiter mindestens einen Teil des optischen Pfads des ersten Strahlenbündels und des zweiten Strahlenbündels umfasst.

5. Das System gemäß Anspruch 2, wobei das System zudem eine Steuerungseinheit (CON) umfasst, wobei die Steuerungseinheit im Betrieb mit der Strahlungsquelle verbunden ist und die optische Energie regelt sowie die ersten Daten bereitstellt, wobei die Steuerungseinheit zudem im Betrieb mit dem Photodetektor verbunden ist und die zweiten Daten empfängt.

6. Das System gemäß Anspruch 5, wobei die Steuerungseinheit einen Regelkreis zum Regeln der optischen Energie (0_P) und/oder der ersten Daten (D_F) ausführt, der mindestens teilweise auf den zweiten Daten (D_F) beruht.

7. Das System gemäß Anspruch 2, wobei das zweite Strahlenbündel (3) von der elektrischen Last des optoelektronischen Geräts (15) abhängig ist.

8. Das System gemäß Anspruch 5 und 7, wobei der Regelkreis die elektrische Last des optoelektronischen Geräts (15) optimiert.

9. Das System gemäß Anspruch 2, wobei das optoelektronische Gerät (15) einen photovoltaischen Wandler umfasst, und wobei das optoelektronische Gerät vorzugsweise einen Festkörperlaser oder eine Leuchtdiode (LED).

10. Das System gemäß Anspruch 2 oder 9, wobei es sich beim optoelektronischen Gerät um ein Gerät mit direktem Bandabstand handelt, vorzugsweise um ein Gerät mit einer einzigen Abzweigung, wobei an der einzigen Abzweigung folgende Schritte durchgeführt werden:
1) Umwandeln des ersten Strahlenbündels in elektrische Energie sowie Empfangen der ersten Daten, und
2) Emittieren des zweiten Strahlenbündels in Richtung des Photodetektors,
wobei die Emission durch das eintreffende ersten Strahlenbündel induziert wird.

11. Das System gemäß Anspruch 2, wobei die optische Wandlerschaltung ausschließlich mit der elektrischen Energie des optoelektronischen Geräts gespeist wird.

12. Das System gemäß Anspruch 2 oder 11, wobei die optische Wandlerschaltung direkt und ohne Spannungsaufwärtswandlung mit der elektrischen Energie des optoelektronischen Geräts gespeist wird.

13. Das System gemäß Anspruch 2, wobei das Gerät eines der folgenden Elemente umfasst:
- einen Temperatursensor,
- einen Drucksensor,
- einen chemischen Sensor,
- einen Ultraschallwandler (CMUT),
- eine Kamera,
- einen Sensor für ionisierende Strahlung (Alpha-, Beta- und/oder Gamma-Strahlung)
- einen Sensor für das elektrische Feld, beispielsweise für EKG-Messungen (Elektrokardiogramm), und/oder
- einen elektrischen Stimulator oder Sensibilisator.

14. Eine Methode zum Versorgen einer optischen Sonde mit elektrischer Energie sowie zum Senden und Empfangen von Daten von der optischen Sonde, wobei die Methode folgenden Schritt umfasst:
- Bereitstellen (S1) eines optischen Sondensystems gemäß einem der Ansprüche 2 bis 13, wobei die Methode zudem folgende Schritte umfasst:
- Emittieren (S2) eines ersten Strahlenbündels von der Strahlungsquelle (6), wobei das erste Strahlenbündel (2) optische Energie (O_P) und erste Daten (D_F) enthält,
- Umwandeln (S3) des ersten Strahlenbündels in elektrische Energie sowie Empfangen der ersten Daten mit dem optoelektrischen Gerät (15),
- Emittieren (S4) des zweiten Strahlenbündels vom optoelektronischen Gerät (15) in Richtung des Photodetektors.

## Revendications

1. Sonde optique (18), la sonde optique pouvant à son extrémité proximale être optiquement connectée à un photodétecteur associé et à une source de rayonnement associée, la sonde comportant un guide optique (8) apte à la connexion de l'extrémité distale à l'extrémité proximale, la sonde optique comportant à son extrémité distale un circuit convertisseur optique (10), ledit circuit comprenant :
un dispositif d'application (20), le dispositif d'application étant conçu pour surveiller et/ou pour manipuler à l'extrémité distale de la sonde, le dispositif d'application étant conçu pour générer des secondes données (D_R) indiquant la fonctionnalité du dispositif d'application, et
un dispositif optoélectronique (15), le dispositif optoélectronique étant conçu pour convertir un premier faisceau de rayonnement (2) en énergie électrique et pour recevoir des premières données comprises dans le premier faisceau de rayonnement, les premières données étant liées à la fonctionnalité du dispositif d'application, le dispositif optoélectronique étant en outre conçu pour émettre un second faisceau de rayonnement (3) vers le photodétecteur associé, le second faisceau de rayonnement comprenant les secondes données,
le dispositif optoélectronique étant conçu, lors de la réception dudit premier faisceau de rayonnement :
pour convertir ledit premier faisceau de rayonnement en énergie électrique et pour recevoir lesdites premières données, et
pour émettre ledit second faisceau de rayonnement vers le photodétecteur associé, ladite émission étant inductible par le premier faisceau de rayonnement entrant, dans lequel le circuit convertisseur optique (10) est alimenté par ladite énergie électrique dans le premier faisceau de rayonnement (2), et le dispositif optoélectronique est conçu pour réaliser une électroluminescence photoinduite (PIEL).

2. Système (100) de sonde optique, le système comprenant :
une source de rayonnement (6) apte à l'émission d'un premier faisceau de rayonnement, ledit premier faisceau de rayonnement (2) comprenant une énergie optique (O_P) et des premières données (D_F),
un photodétecteur (5), le photodétecteur étant conçu pour détecter un second faisceau de rayonnement (3), et
une sonde optique (18) selon la revendication 1, dans lequel la sonde optique est à son extrémité proximale connectée optiquement au photodétecteur et à la source de rayonnement.

3. Système selon la revendication 2, dans lequel le guide optique (8) est conçu pour guider ledit premier faisceau de rayonnement (2) de l'extrémité proximale à l'extrémité distale, et étant en outre conçu pour guider ledit second faisceau de rayonnement (3) de l'extrémité distale à l'extrémité proximale, le premier faisceau de rayonnement et le second faisceau de rayonnement étant conçus pour être guidés le long du même chemin optique, ou d'un chemin optique parallèle, dans ledit guide optique.

4. Système selon la revendication 3, dans lequel le guide optique comprend une fibre optique, la fibre optique comprenant au moins une partie dudit chemin optique pour le premier faisceau de rayonnement et le second faisceau de rayonnement.

5. Système selon la revendication 2, dans lequel le système comprend en outre une unité de commande (CON), l'unité de commande étant connectée de manière fonctionnelle à la source de rayonnement et conçue pour commander l'énergie optique et pour fournir les premières données à celle-ci, l'unité de commande étant en outre connectée de manière fonctionnelle au photodétecteur et conçue pour recevoir les secondes données à partir de celui-ci.

6. Système selon la revendication 5, dans lequel l'unité de commande est conçue pour faire fonctionner une boucle de commande pour commander l'énergie optique (O_P) et/ou les premières données (D_F) en fonction, au moins partiellement, des secondes données (D_F).

7. Système selon la revendication 2, dans lequel ledit second faisceau de rayonnement (3) dépend d'une charge électrique sur le dispositif optoélectronique (15) .

8. Système selon les revendications 5 et 7, dans lequel ladite boucle de commande est conçue pour optimiser la charge électrique sur le dispositif optoélectronique (15).

9. Système selon la revendication 2, dans lequel le dispositif optoélectronique (15) comprend un convertisseur photovoltaïque, de préférence le dispositif optoélectronique comprend un laser à solide ou une diode électroluminescente (DEL).

10. Système selon la revendication 2 ou 9, dans lequel le dispositif optoélectronique est un dispositif à bande interdite directe, de préférence un dispositif à jonction unique,
1) la conversion dudit premier faisceau de rayonnement en énergie électrique et la réception desdites premières données, et
2) l'émission dudit second faisceau de rayonnement vers le photodétecteur, ladite émission étant inductible par le premier faisceau de rayonnement entrant, étant effectuées au niveau de ladite jonction unique.

11. Système selon la revendication 2, dans lequel le circuit convertisseur optique peut être alimenté uniquement par ladite énergie électrique provenant du dispositif optoélectronique.

12. Système selon la revendication 2 ou 11, dans lequel le circuit convertisseur optique peut être alimenté directement par ladite énergie électrique provenant du dispositif optoélectronique sans aucune conversion montante de la tension.

13. Système selon la revendication 2, dans lequel le dispositif d'application comprend :
un capteur de température, et/ou
un capteur de pression, et/ou
un capteur chimique, et/ou
un transducteur à ultrasons (CMUT), et/ou une caméra, et/ou
un capteur de rayonnement ionisant (alpha, bêta et/ou gamma), et/ou
un capteur de champ électrique, par exemple, pour mesurer un ECG (électrocardiogramme), et/ou un électrostimulateur ou sensibilisateur.

14. Procédé d'alimentation d'une sonde optique en énergie électrique et d'envoi et de réception des données de la sonde optique, le procédé comprenant :
la fourniture (S1) d'un système de sonde optique selon l'une quelconque des revendications 2 à 13, ledit procédé comprenant en outre :
l'émission (S2) d'un premier faisceau de rayonnement à partir de la source de rayonnement (6), ledit premier faisceau de rayonnement (2) comprenant une énergie optique (O_P) et des premières données (D_F),
la conversion (S3) au niveau du dispositif optoélectronique (15) dudit premier faisceau de rayonnement en énergie électrique et la réception desdites premières données,
l'émission (S4) dudit second faisceau de rayonnement depuis le dispositif optoélectronique (15) vers le photodétecteur.
